# EUROPEAN PATENT APPLICATION

(11) **EP 1 847 277 A1**
(43) Date of publication of application: **24.10.2007**
(21) Application number: 06711538.6
(22) Date of filing: 11.01.2006
(51) Int. Cl.: A61L 27/00

(54) **SHEET-SHAPED COMPOSITION UTILIZING AMNION AND METHOD OF PREPARING THE SAME**

(30) Priority: 14.01.2005 JP 2005008381
(71) Applicant: ArBlast Co., Ltd., Kobe Hyogo 650-0047 (JP)
(72) Inventor: KURIHARA, Eiji c/o ArBlast Co., Ltd., Kobe International Business, Kobe-city Hyogo 6500047 (JP); HAMURO, Junji, awa, 2410813 (JP); NAKAMURA, Takahiro, Kyoto, 6040951 (JP)
(74) Representative: Müller Fottner Steinecke
(86) International application number: PCT/JP2006/300189
(87) International publication number: WO 2006/075602

(57) **Abstract**

A sheet-shaped composition that is applicable to a wide spectrum of uses and can be transplanted through simple and easy transplanting technique. There is provided a sheet-shaped composition comprising amniotic membrane and, attached to the surface thereof, fibrinogen and thrombin. In one form, a cell layer is formed on the amnion on its side opposite to the side of adhesive component attachment.

## Description

### TECHNICAL FIELD

The present invention relates to a sheet-shaped composition using amniotic membrane and a method of producing the same. The sheet-shaped composition of the present invention can be used as, for example, a transplantation material for reconstructing the ocular surface or the skin.

### BACKGROUND ART

Recently, a technology of regeneration medicine for corneal epithelial diseases by using corneal epithelial stem cells has been made into practice. Other than corneal epithelial diseases, new attempts of cell culture have been also carried out actively for the purpose of treating various diseases such as endothelial diseases and retinal diseases in the field of ophthalmology and epidermal diseases in the field of dermatology. Demands for the regenerative medicine are likely to increase in the future. One of supports for cell culture used in regenerative medicine is amniotic membrane. The amniotic membrane is a tissue existing in the innermost side of the placenta in the mammalian uterus and is made of a single layer membrane enclosing a fetus. The amniotic membrane is known to be extremely excellent culture substrate for various cells. To date, cultured cell sheets using amniotic membrane as a carrier, for example, a cultured corneal epithelium cell sheet, a cultured oral mucosal epithelial sheet, and a cultured epidermal cell sheet have been produced (see, for example, patent documents 1 to 3).

[Patent document 1] International Publication No. WO 03/043542 A1, pamphlet
[Patent document 2] International Publication No. WO 03/092762 A1, pamphlet
[Patent document 3] International Publication No. WO 2004/078225 A1, pamphlet

### DISCLOSURE OF THE INVENTION

### [Problems to be Solved by the Invention]

In order to allow a cultured cell sheet or an amniotic membrane sheet to have high therapeutic effect, it is necessary that such a sheet adhere to a transplantation site rapidly and that the adhesion state be maintained. That is to say, such transplantation sheets are required to have high adhesive property and survival property. However, under the present circumstances, since sufficient adhesive force cannot be obtained, when a cultured cell sheet or an amniotic membrane sheet is transplanted to a living body, in almost all the cases, the sheet needs to be sutured. In general, when the sheet is sutured, the following inconveniences occur: 1) the tissue is mechanically damaged, which leads to necrosis of cells; 2) bacterial infection is induced, and 3) capillary vessels are damaged so as to cause bleeding. When necrotic cells or bacteria are generated, a living body tries to remove them, and cells of the immune system such as Langerhans cells, dendritic cells, macrophages and leukocytes, and the like, are activated, so that inflammation is elicited. Furthermore, from the macrophage, a growth factor is released and angiogenesis is caused. Inflammation may cause congestion, watery eye, gum, fever, flare, itching, and the like. The angiogenesis in the retina site may lead to the deterioration of visual acuity. Such physiological effect not only reduces the original therapeutic effect but also requires additional treatment, resulting in bringing much inconvenience in the clinical site. These have been a large problem when the cultured cell sheet or an amniotic membrane sheet is transplanted to a living body. Meanwhile, suturing is a very complicated operation and needs experienced and skilled persons. Therefore, under current circumstances, persons and institution capable of carrying out sheet transplantation are very limited, which has delayed further spreading the regenerative medicine. Furthermore, since it takes extra time and labor to carry out suturing, accordingly burden to a patient is increased, so that the state of the patient may be deteriorated. In order to solve such problems, when a cultured cell sheet or an amniotic membrane sheet is transplanted to a living body, it is desirable that suturing is not necessary.
On the other hand, as a material used for adhesion to a living body without suturing, to date, fibrin glue that is a tissue adhesive (Tiseel^{™} (Baxter), Bolheal^{™} (KAKETSUKEN), and Beriplast^{™} (Aventis Pharma)) have been mainly used. The fibrin glue is an agent for adhering and closing the tissue by imitating the final stage of the blood coagulation in a living body. When a cultured cell sheet or an amniotic membrane sheet adhere to a living body by using fibrin glue, an operator has to purchase additionally a fibrin glue and prepare it during an operation. However, it takes a long time and much labor to prepare fibrin glue. Since the fibrin glue is easy to be solidified, it is difficult to handle it. The fibrin glue is not easily used as an adhesive to allow a sheet to adhere to a tissue.
Other than fibrin glue, a product for allowing a material to adhere to a living body without suturing includes Taco Comb^{™} (Nycomed Pharma). This product is used as an injury covering agent and is a pharmaceutical agent using a collagen sheet as a carrier and including adhesives fixed on one side thereof. Since the product includes an adhesive applied thereto, time and labor for preparation is not necessary. Thus, only by applying Taco Comb to a suffered site, adhesion to the tissue is completed. However, a material used as a carrier of Taco Comb is a chemical material produced by reconstructing collagen. Therefore, it does not allow a live tissue of biological origin, for example, an amniotic membrane sheet or a cultured cell sheet to adhere as it is to a living body. Furthermore, a sheet-shaped graft constructed using this product has a thickness of several mm, so that transparency is poor. Therefore, it is never applicable to a case that requires sufficient transparency after the transplantation (for example, reconstruction of the ocular surface). Furthermore, in the reconstruction of epithelial tissue represented by the reconstruction of the ocular surface, a transplantation site has little physical space, and therefore a graft material needs to be thin. Also from this viewpoint, the above-mentioned product is not appropriate.
As mentioned above, to date, there has not been provided a technology capable of allowing a functional tissue of biological origin to adhere to a living body without suturing.
Under such circumstances, the present invention has an object to provide a sheet-shaped composition that is applicable to a wide spectrum of uses, for example, reconstruction of the ocular surface, reconstruction of the skin or the epidermis, covering of thermal injury, and the like, and that can be transplanted by a simple and easy transplanting technique. In particular, the present invention has an object to provide a sheet-shaped composition that is suitably applicable to the uses requiring high transparency.

### [Means to Solve the Problems]

In order to achieve the above-mentioned object, the present inventors have used amniotic membrane as a support in constructing a sheet-shaped composition. In order to improve an adhesive property and a survival property after transplantation, fibrin and thrombin have been applied to the surface of the amniotic membrane. The resultant sheet-shaped composition is very thin and can secure high transparency. Furthermore, the resultant sheet-shaped composition has sufficient strength. Meanwhile, in order to examine the adhesive property and the survival property, the sheet-shaped composition was applied to the ocular surface. As a result, rapid adhesion was observed. Furthermore, the favorable adhesive state was maintained for a long time. As mentioned above, the sheet-shaped composition configured by attaching fibrin and thrombin on the surface of the amniotic membrane is excellent in transparency, strength, as well as adhesive property and survival property. That it to say, it was found that the sheet-shaped composition met the requirements for a wide spectrum of uses. Furthermore, since the sheet-shaped composition has high adhesive property and survival property, it is thought that it can realize a simple and easy transplantation technique without carrying out suturing. When suturing is not necessary, the problems caused by suturing, for example, elicitation of inflammation and occurrence of angiogenesis are dissolved. Furthermore, time and labor necessary for transplantation to a living body can be omitted. The number of operators who can carry out sheet transplantation increase, which is expected to contribute the development of regenerative medicine. Meanwhile, since the resultant sheet-shaped composition has high transparency, it can be used for the application that requires high transparency by itself, in particular, for reconstructing the ocular surface, and the like.
The present inventor have obtained the above-mentioned findings, and further examined the adhesive property of the above-mentioned sheet-shaped composition. As a result, the present inventors have successfully found a relationship between the adhesive property and the addition amount (attached amount) of fibrinogen and thrombin that are adhesive components. With this finding, since the necessary adhesive property can be maintained and the addition amount of fibrinogen and the like can be reduced, the angiogenesis due to the effect of fibrinogen and the like after transplantation can be minimized. Therefore, in the uses requiring to suppress the inflammation and angiogenesis after transplantation, for example, in the reconstruction of the ocular surface, extremely preferable transplantation material can be provided.

The present invention is based on the above-mentioned findings and has the following configurations.
A sheet-shaped composition comprising amniotic membrane having fibrinogen and thrombin attached on the surface thereof. In one embodiment of the present invention, the surface of the amniotic membrane on which fibrinogen and thrombin are attached is in a dry state. In another embodiment of the present invention, substantially all the surface of amniotic membrane is in a dry state.
Furthermore, in one embodiment of the present invention, on the surface of the amniotic membrane, aprotinin is attached in addition. The use of aprotinin in combination enables suppressing the decomposition of a fibrin clot and maintaining or strengthening the adhesive force.
As the amniotic membrane, amniotic membrane from which the epithelium has been removed may be used. When epithelial components are not contained, a sheet-shaped composition with less immunogenicity can be obtained.
In a further embodiment of the present invention, a cell layer made of cells of biological origin is formed on the amniotic membrane, and the opposite side to the surface on which the cell layer is formed is a surface to which fibrinogen and other components are to be attached. The cultured cell sheet in this embodiment becomes a transplantation sheet having high therapeutic effect by the effect of the contained cells. The examples of cells forming the cell layer include, for example, cells derived from corneal epithelium, conjunctival epithelium, skin epidermis, hair follicle epithelium, oral mucosal epithelium, iris pigment epithelium, retina pigment epithelium, respiratory tract mucosa epithelium or intestinal tract mucosa epithelium.
It is preferable that the amount of fibrinogen attached to the amniotic membrane is 0.5 mg to 20 mg per 1 cm² of amniotic membrane. Similarly, it is preferable that the amount of thrombin attached to the amniotic membrane is 1 µg to 1 mg per 1 cm² of amniotic membrane. This is advantageous because a high adhesive property can be maintained as well as inflammation and angiogenesis after transplantation can be reduced.
The sheet-shaped composition of the present invention can be prepared to be 10 µm to 200 µm in thickness by using amniotic membrane.
In the sheet-shaped composition of the present invention, by attaching thrombin and the like in advance, an adhesive property with respect to human tissue can be obtained and excellent adhesive force can be obtained without suturing in transplantation.

On the other hand, the present invention also provides a method of producing the above-mentioned sheet-shaped composition. Specifically, the following configurations are provided.
That is to say, a method of producing a sheet-shaped composition, comprising : (a) a step of preparing amniotic membrane; and (b) a step of attaching fibrinogen and thrombin to the surface of the amniotic membrane.
In one embodiment of the present invention, after the step (b), a drying step (step (c)) is carried out. The drying process is carried out to, for example, an entire composition or only a surface to which fibrinogen and other components are attached, depending upon the uses.
In one embodiment of the present invention, the step (step b-1) of attaching aprotinin to the surface of the amniotic membrane is carried out. Note here that it is preferable that this step is carried out simultaneously with the attaching step of fibrinogen and thrombin.
As a part of the step (a), it is preferable that a step (step a-1) of removing the epithelium from the amniotic membrane is carried out. In this embodiment, a sheet-shaped composition is produced by using amniotic membrane from which the epithelium has been removed.
Furthermore, as a part of the step (a), a step (step a-2) of drying amniotic membrane can be carried out. That is to say, in this embodiment, during the process of producing the sheet-shaped composition, the amniotic membrane is once dried so as to be subjected to the later process.
In a further embodiment of the present invention, between the step (a) and the step (b), a step (step A) of forming a cell layer made of cells of biological origin on the amniotic membrane is carried out. With this step, a cell layer is formed on the amniotic membrane and finally a cultured cell sheet is produced.
Hereinafter, the configuration of the present invention is described in more detail.

### BRIEF DESCRIPTION OF THE DRAWINGS

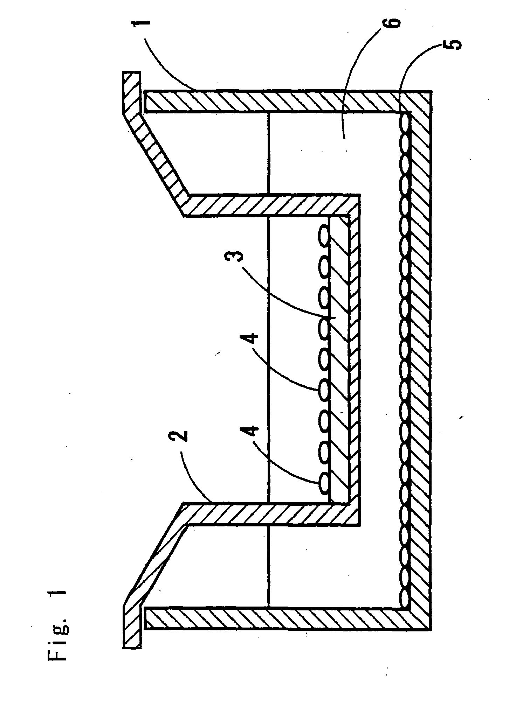

Fig. 1 is a cross-sectional view schematically showing a state of an instrument, etc. when oral mucosal epithelial cells and corneal epithelial cells are cultured on amniotic membrane. In a culture dish 1, a culture insert 2 is placed. On the bottom surface of the culture dish 1, a 3T3 cell layer 5 is formed. Furthermore, on the bottom surface of the culture insert 2, amniotic membrane 3 is placed, and oral mucosal epithelial cells and corneal epithelial cells 4 are cultured thereon. Reference numeral 6 denotes a culture medium.

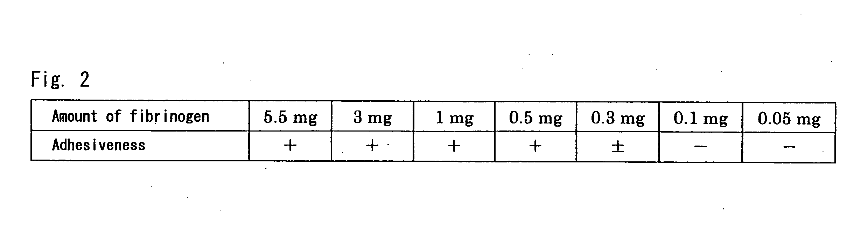

Fig. 2 is a table summarizing experiment results as to the relationship between the adhesion amount of adhesive components and the adhesive property. The line "adhesion or not" is represented by + (sheet is neither shifted nor peeled off) and - (sheet is shifted).

### REFERENCE MARKS IN THE DRAWINGS

- 1: culture dish (first container)
- 2: culture insert (second container)
- 3: amniotic membrane
- 4: corneal epithelial cells
- 5: 3T3 cell layer
- 6: medium

### BEST MODE OF CARRYING OUT THE INVENTION

The first aspect of the present invention relates to a sheet-shaped composition. In the sheet-shaped composition of the present invention, amniotic membrane is used as one of the main components. Thanks to the high transparency and strength of the amniotic membrane, a sheet-shaped composition that is excellent in the transparency and strength can be configured. Furthermore, the sheet-shaped composition is further excellent in biological affinity and low immunogenicity as amniotic membrane is high in biological affinity and low in immunogenicity. With the use of amniotic membrane, effects such as an anti-inflammation effect, suppression of the formation of trace, and inhibition of angiogenesis can be expected. It is preferable to use amniotic membrane from the viewpoint that a cell layer is formed well when the sheet-shaped composition of the present invention includes a cell layer. That is to say, as mentioned below, the sheet-shaped composition having a cell layer is formed by seeding and culturing cells of interest on amniotic membrane as a substrate (support medium). Since the amniotic membrane has a property that cells excellently adhere and proliferate thereon, the use of amniotic membrane enables excellent adhesion and proliferation of the cells and formation of a cell layer.
It is further preferable to use amniotic membrane from which the epithelium has been removed by, for example, scraping treatment. This is advantageous because when epithelial components are not contained, a sheet-shaped composition with less immunogenicity can be obtained. Furthermore, advantages in terms of manufacture that on the amniotic membrane from which the epithelium has been removed, cells adhere and proliferate further excellently, allowing to construct a corneal epithelium-like sheet with high quality in a shorter time.
Whether or not the epithelium has been removed from the amniotic membrane can be determined by examining the presence of cells of the amniotic membrane epithelium layer in the sheet-shaped composition. Note here that it is particularly preferable that human amniotic membrane is used as amniotic membrane.

In the sheet-shaped composition of the present invention, on the surface of amniotic membrane, fibrinogen and thrombin (hereinafter, which are also collectively referred to as "adhesive components"). Thus, when the sheet-shaped composition of the present invention is transplanted, firstly, fibrinogen is specifically hydrolyzed by thrombin so as to form fibrin, and then, fibrins are polymerized so as to form a stable fibrin clot which exhibits an adhesive effect.
Fibrinogen and thrombin are attached to one side or both sides of the amniotic membrane depending upon uses of the sheet-shaped composition of the present invention. When they are attached to one side of the amniotic membrane, regardless of the presence or absence of the epithelium of the amniotic membrane, a surface at the side of the chorionic membrane of the amniotic membrane (that is to say, a surface that is opposite side to the epithelium side) becomes an attaching surface. Therefore, when a sheet-shaped composition having such a configuration is used, the amniotic membrane is to be transplanted on an application site in a state in which the surface at the side on which the epithelium of the amniotic membrane has been existed is allowed to face upward. When the sheet-shaped composition is transplanted in the living body for the purpose of biologically adhesion, it is appropriate to attach adhesive components on both sides of the amniotic membrane.

Note here that as mentioned below, the sheet-shaped composition of the present invention is prepared in an appropriate state (for example, dry state or wet state) through a step of attaching fibrinogen and thrombin to the surface of amniotic membrane by considering the intended uses. Therefore, fibrin is expected to be generated from a part of fibrinogen before the sheet-shaped composition is used depending upon the state during process and/or the final state. Therefore, the sheet-shaped composition of the present invention may include fibrin or a fibrin clot generated by such a reason.

The origin of the fibrinogen and thrombin is not particularly limited. The fibrinogen and thrombin can be prepared by using blood of, for example, human, monkey, chimpanzee, bovine, horse, sheep, pig, and the like, as a starting material. Furthermore, as the fibrinogen and thrombin, a recombinant obtained by using cultured cells (for example, CHO cells or COS cells) may be used. It is preferable to use fibrinogen and thrombin derived from human (in particular, human-derived recombinant). This is advantageous from the viewpoint of safety including immunogenicity. Furthermore, by considering the stable quality and problem of infection, it is particularly preferable to use a recombinant.
It is particularly preferable to use fibrinogen and thrombin derived from blood of a patient (recipient) who is going to be subjected to transplantation of the sheet-shaped composition of the present invention. This is advantageous because immunological rejection may not be induced.
Note here that the origins of the fibrinogen and thrombin may not necessarily be the same. For example, the combination of fibrinogen derived from human blood and thrombin derived from bovine blood may be used..

The attached amount of fibrinogen and thrombin is not particularly limited. For example, the attached amount of fibrinogen can be set in the range from 0.1 mg to 50 mg per 1cm² of amniotic membrane. Similarly, the attached amount of thrombin can be set in the range from 0.5 µm to 10 mg per 1cm² of amniotic membrane.
Adhesive force is primarily considered when setting the attached amount of fibrinogen and thrombin. That is to say, in order to obtain the necessary adhesion force, the attached amount of these components need to be set. On the other hand, when the attached amount of fibrinogen and thrombin is too large, immune reaction or angiogenesis may tend to be induced, although depending upon the origin of fibrinogen to be used.
Herein, in a case that a sheet-shaped composition applied to reconstruction of the ocular surface(for example, when angiogenesis due to these components after transplantation may occur) in order to suppress the induction of the angiogenesis as much as possible, it is preferable that the attached amount of these components is reduced. By setting the attached amount of these components as small as possible, the angiogenesis after transplantation can be suppressed and high therapeutic effect can be expected. As described in the below mentioned example, as a result of the investigation by the present inventors, when the attached amount of fibrinogen is about 0.5 mg or more per 1 cm² of amniotic membrane, the excellent adhesive force with respect to the ocular surface was observed. As to thrombin, even when the attached amount of thrombin is 1 µg per 1 cm² of amniotic membrane, the excellent adhesive force with respect to the ocular surface was observed. Based on these findings, the preferable range of the attached amount of fibrinogen is 0.5 mg to 20 mg per 1 cm² of amniotic membrane. Further preferable range is 0.5 mg to 10 mg per 1 cm² of amniotic membrane. More preferable range is 0.5 mg to 6 mg (specifically, for example, about 0.5 mg, about 1 mg, and about 2 mg) per 1 cm² of amniotic membrane. Similarly, the preferable range of the attached amount of thrombin is 1 µg to 1 mg per 1 cm² of amniotic membrane. Further preferable range is 5 µg to 200 µg per 1 cm² of amniotic membrane. More preferable range is 10 µg to 100 µg (specifically, for example, about 10 µg, about 20 µg, and about 30 µg) per 1 cm² of amniotic membrane.

In one embodiment of the present invention, in addition to fibrinogen and thrombin, aprotinin is attached to the surface of amniotic membrane. Aprotinin inhibits the fibrin clot formed by the effect of thrombin from being dissolved by plasmin. Therefore, by using aprotinin together, the decomposition of the fibrin clot can be suppressed. As a result, the adhesive force can be maintained or reinforced.
The origin of aprotinin is not particularly limited. The aprotinin derived from the pancreas of, for example, bovine, horse, sheep, pig, monkey, chimpanzee, and the like. Furthermore, a recombinant aprotinin obtained by using cultured cells (for example, CHO cells or COS cells) may be used. It is preferable to use a recombinant from the viewpoint of the stable quality and problem of infection.
When aprotinin is used, the attached amount thereof is not particularly limited. For example, the attached amount of aprotinin can be set in the range from 0.1 KIU to 200 KIU per 1 cm² of amniotic membrane. We examined how a change in the attached amount of aprotinin affects the adhesion force in addition to the investigation of the above-mentioned attached amount of fibrinogen. As a result, even when the amount of aprotinin is set in the range from 1 KIU to 2 KIU, a sufficient adhesive force to the ocular surface was observed. Based on this finding, the preferable range of the attached amount of aprotinin is 1 KIU to 100 KIU per 1 cm² of amniotic membrane. Further preferable range is 1 KIU to 20 KIU per 1 cm² of amniotic membrane. More preferable range is 1 KIU to 10 KIU (specifically, for example, about 1 KIU, about 2 KIU, and about 3 KIU) per 1 cm² of amniotic membrane. When the amount of the aprotinin is too large, the manufacturing cost is increased and furthermore, the side effect caused by the immunogenicity, etc. of the aprotinin itself may be increased. On the other hand, when the amount of aprotinin is too small, the effect of aprotinin of suppressing the deposition of fibrin clot may not be exhibited sufficiently.
In various purposes, the fibrin clot is used as an adhesive, generally with aprotinin. As a result of the investigation by the present inventors, in the sheet-shaped composition of the present invention, even if aprotinin is not used, it has been found that sufficient adhesive force with respect to a living body can be obtained. When aprotinin may not be used, a configuration can be simplified, so that advantages in terms of manufacture and cost can be achieved. In addition, it becomes unnecessary to consider the side effect caused by the immunogenicity etc. of the aprotinin itself.

The sheet-shaped composition in accordance with one embodiment of the present invention includes a cell layer which is formed on the amniotic membrane. In this embodiment, adhesive components (for example, fibrinogen) are attached on the side of the amniotic membrane on which a cell layer is not formed.
In this embodiment, amniotic membrane from which the epithelium has been removed is used. Then, at the side where the epithelium has been present, a cell layer is formed. This cell layer is formed from cells of biological origin. The origin of the cells constituting the cell layer is not particularly limited. Examples of the cells include cells derived from corneal epithelium, conjunctival epithelium, skin epidermis, hair follicle epithelium, oral mucosal epithelium, iris pigment epithelium, retina pigment epithelium, respiratory tract mucosa epithelium or intestinal tract mucosa epithelium, and the like. A cell layer may be formed by using two types or more of cells that are different from each other. Formation from two types or more of cells that are derived from different origins is also referred to as "hybridization" in this specification. The form in which cells are contained in the hybridized cell layer (state of hybridization) is not particularly limited and, for example, cells may be dispersed or some cells (or plural types of cells) may be present as a group. Furthermore, the content of cells may not be uniform over the entire cell layer. The cell layer may be a single layer or multilayer (stratified layers).

When the hybridized cell layer is provided, types of cells constituting the cell layer are described hereinafter, by taking a case where the sheet-shaped composition of the present invention is constructed for the corneal epithelium.
The hybridized cell layer contains two types or more of cells. One type of cells are referred to as first cells and the other type of cells that are different from the first cells are referred to as second cells for convenience of explanation. Firstly, autologous cells are used as the first cells. In this specification, "autologous" indicates a subject to whom the sheet-shaped composition of the present invention is to be applied, that is, a subject who undergoes transplantation (recipient). On the other hand, other than such "autologous" is referred to as "allogeneic." The type of the first cells is not particularly limited as long as the first cells can form a corneal epithelium-like mucosal epithelium layer when they are hybridized with the below-mentioned second cells. Examples of the first cells include cells derived from mucosal epithelium such as oral mucosal epithelium, conjunctival epithelium, and nasal mucosal epithelium, or cells derived from undifferentiated cells capable of constructing such mucosal epithelium (that is, mucosal epithelium stem cells). Herein, the term "derived from or of origin" is used for the purpose of specifying a starting material. Therefore, for example, cells derived from (of origin of) the oral mucosal epithelium indicates cells obtained by using oral mucosal epithelial cells as a starting material. Furthermore, in the present invention, the term "undifferentiated cells capable of constructing such mucosal epithelium" indicates cells having the potency of differentiating into cells constituting mucosal epithelium. For example, undifferentiated cells capable of constructing oral mucosal epithelium indicates cells capable of differentiating into oral mucosal epithelial cells. Specific examples of the undifferentiated cell include a precursor cell or a stem cell of cells constituting specific tissue, for example, oral mucosal epithelium or conjunctival epithelium, and the like, or an epithelial stem cell with lower differentiation.

The hybridized cell layer may include two or more different types of the first cells. For example, a cell layer may be constructed from cells derived from oral mucosal epithelium and cells derived from conjunctival epithelium.

The "oral mucosal epithelium" in the present invention may include oral crevicular mucosal epithelial part, labial part, palate part, buccal part, and the like. Whether or not the cells are derived from oral mucosal epithelium can be confirmed by using, as an indicator, the expression of keratin 4 or keratin 13 specific to oral mucosal epithelium. Alternatively, the expression of keratin 3 can be used as an indicator. This keratin 3 is known to be one of the cornea-specific keratins. However, keratin 3 was reported to be expressed also in the oral mucosal epithelial cell. Note here that it is preferable that oral mucosal epithelial cells are used as a material for producing a composition for transplantation of corneal epithelium from a viewpoint that it expresses this cornea-specific keratin, keratin 3.
On the other hand, by examining the expression of genes specific to an oral mucosal epithelial cell, it can be confirmed that cells are derived from oral mucosal epithelium.
Similarly, in the case of cells derived from a tissue other than oral mucosal epithelium, by examining the expression of the marker or gene specific to the tissue, the origin thereof can be confirmed.

Specific examples of the second cells include cells derived from corneal epithelium, conjunctival epithelium or amniotic membrane epithelium. Among them, it is preferable that the second cells are cells derived from corneal epithelium or conjunctival epithelium. This is advantageous because the cell layer constructed by cells derived from ocular surface tissue can have a property closer to that of corneal epithelium. It is particularly preferable that the second cells are derived from corneal epithelium. This is advantageous because a layer that is more similar to corneal epithelium can be obtained.
The second cells may be autologous cells or allogeneic cells. When autologous cells are used, a cell layer with no or little problem of immunological rejection can be obtained. When allogeneic cells are used, since it is easy to prepare cells, it is advantageous from the viewpoint of manufacturing. The cell layer of the present invention may include two or more different types of second cells. For example, the cell layer may be constructed in a state which includes, for example, cells derived from corneal epithelium and cells derived from conjunctival epithelium.

Whether or not the cell layer in the sheet-shaped composition of the present invention includes cells that are derived from the corneal epithelium can be confirmed by using, as an indicator, the expression of keratin 3 or keratin 12 specific to corneal epithelium. Alternatively, the expression of keratin 4 can be used as an indicator.
Similarly, in the case of cells derived from the tissue other than corneal epithelium, by examining the expression of the marker or gene specific to the tissue, the origin thereof can be confirmed.

Since the sheet-shaped composition of the present invention employs amniotic membrane as a support, it can be constructed very thinly. When the sheet-shaped composition of the present invention does not include a cell layer, the sheet-shaped composition can be prepared to the thickness, for example, in the range from 10 µm to 100 µm. When the sheet-shaped composition includes a cell layer, it can be prepared to the thickness, for example, in the range from 20 µm to 200 µm. Thus, very thin state is also one of the main features of the present invention. With this feature, the composition becomes applicable to a wide spectrum of uses. In particular, making the most of the high transparency, it can be applied to reconstruction of the ocular surface.

The state of the sheet-shaped composition of the present invention is not particularly limited and it may be any of a dry state (for example, a lyophilization state), a semidry state, and a wet state (for example, a state in which it is dipped in a solution). For example, only a surface of the amniotic membrane on which an adhesive components are attached may be a dry state, or entire amniotic membrane may be in a dry state. When a cell layer is included, it is preferable that the cell layer part is in a wet state and the other parts may be in, for example, a semidry or a dry state. Specifically, for example, the cell layer is made in a wet state and a surface of amniotic membrane on which fibrinogen and the like (including fibrinogen and the like) is attached may be in a dry or a semidry state and the other parts of the amniotic membrane may be in a wet state.
The sheet-shaped composition of the present invention can be provided in a state in which it is contained in a case such as glass case or plastic case, or covered with a transparent film or light shielding sheet. Preferably, the sheet-shaped composition of the present invention is provided in a sealing state. Furthermore, in general, it is provided in a state in which sterilization treating is carried out in advance.

Instead of amniotic membrane, a collagen sheet having the same properties (thickness, transparency, and the like) as those of amniotic membrane may be used so as to construct a sheet-shaped composition. Since amniotic membrane is rich in the type IV collagen, examples of the above-mentioned collagen sheet include a collagen sheet which is rich in collagen IV. The sheet can be constructed by using, for example, bovine, pig, or horse collagen.

The sheet-shaped composition of the present invention is used as a transplantation material and the like for reconstructing the tissue. The application fields of the sheet-shaped composition of the present invention can include the fields of ophthalmology, digestive surgery, gynecology, and dermatology. Examples of application sites, diseases to be treated, and uses in each field are described hereafter.

### 1. Field of ophthalmology

The sheet-shaped composition without a cell layer can be applied to reconstruction of the sclera and the cornea (treatment for pterygium, the corneal epithelium deficiency, and the like), and to prevention of symblepharon. On the other hand, the sheet-shaped composition including a cell layer can be applied to reconstruction of the cornea or the retina (treatment for Stevens-Johnson syndrome, chemical injury and burn, ocular pemphigoid, ablation of retina, aging macular degeneration, glaucoma, retinitis pigmentosa, and the like).

### 2. Field of dermatology

The sheet-shaped composition without a cell layer can be applied to dress cutaneous ulcer and burn. On the other hand, the sheet-shaped composition including a cell layer can be applied to treat epidermis-diabetic ulcer (epidermis), bullous epidermolysis, or burn.

Another aspect of the present invention relates to a method of producing a sheet-shaped composition. The production method of the invention includes the following steps: (a) preparing amniotic membrane; and (b) attaching fibrinogen and thrombin to the surface of amniotic membrane.
"Amniotic membrane" is a membrane covering the outermost layer of the uterus and the placenta in mammals, and including a basal membrane and an epithelium layer formed on parenchymal tissue rich in collagen. It is preferable that human amniotic membrane is used as amniotic membrane. Human amniotic membrane can be collected by, for example, human embryonic membrane, placenta, etc. obtained at the time of afterbirth at delivery. Specifically, the human amniotic membrane can be prepared by treating and purifying the integrated material including human embryonic membrane, placenta, and umbilical cord obtained right after delivery. The treating and purifying method can employ a well-known method, for example, a method described in Japanese Patent Unexamined Publication No. H5-5698, etc. That is to say, amniotic membrane is detached from the embryonic membrane obtained at delivery and remaining tissue is removed by a physical treatment such as ultrasonic cleansing and an enzyme treatment, and the like. Then, appropriate washing process is carried out and thus the human amniotic membrane can be prepared.
The thus prepared human amniotic membrane can be cryopreserved before use. The human amniotic membrane can be frozen in a liquid mixing equal volume ratio of DMEM (Dulbecco's modified Eagle's medium) and glycerol at, for example, -80°C. By cryopreservation, not only the improvement in operation property but also reduction of the antigenicity can be expected.

Intact amniotic membrane may be used but it is preferable that amniotic membrane from which epithelium has been removed by a scraping treatment, etc. is used. By removing the epithelium, antigenicity is reduced. For example, cryopreserved human amniotic membrane is thawed and then subjected to a treatment with EDTA or proteolytic enzyme so as to loosen the adhesion between cells. Then, epithelium is scraped by using a cell scraper, etc. Thus, the human amniotic membrane from which epithelium has been removed can be prepared.

It is preferable that the amniotic membrane has been subjected to drying process in advance. By using a dried amniotic membrane, adhesive components such as fibrinogen can be attached excellently. The drying process herein can include, for example, lyophilization, air drying, vacuum drying, and drying under reduced pressure. Among them, it is preferable to employ lyophilization. This is advantageous because lyophilization may not easily reduce the flexibility of amniotic membrane.

Attachment of fibrinogen and thrombin to the surface of amniotic membrane may be carried out separately or simultaneously. The attaching method is not particularly limited. Example of the attaching method include a method of applying, dropping or spraying a solution containing components to be attached onto the surface of amniotic membrane, or a method of immersing amniotic membrane into a solution containing components to be attached. Furthermore, fibrinogen itself (or thrombin itself) or fibrinogen (or thrombin) is dissolved in an appropriate solution, and then the deposited components are added (dredged) to the surface of amniotic membrane. Thereby, fibrinogen (or thrombin) is allowed to be attached to the surface of amniotic membrane.
Preferably, a mixed solution containing these two components is prepared, and this mixed solution is, for example, applied or dripped so as to attach fibrinogen and thrombin onto the surface of amniotic membrane simultaneously. A specific example of the method of attaching such two components simultaneously is described hereafter.
Firstly, a fibrinogen solution is prepared. Specifically, fibrinogen is dissolved in a solvent such as ethanol (for example, 94% ethanol) so that the desired concentration is obtained. Instead of ethanol, alcohols such as dehydrated ethanol, isopropanol and methanol, and acetone, or the like, can be used as a solvent. On the other hand, by the same procedure, a thrombin solution is separately prepared. As the solvent in this case, for example, ethanol (for example, 99.5% ethanol), alcohol such as dehydrated ethanol, isopropanol and methanol, and acetone, and the like, can be used.
Next, the fibrinogen solution and the thrombin solution, which have been prepared by the above-mentioned procedure, are mixed. As mentioned above, application, dropping and the like on amniotic membrane are carried out by using the thus obtained mixed solution. As mentioned above, when the fibrinogen solution and the thrombin solution are mixed, and the mixed solution is used so as to carry out attachment operation, it is preferable to prevent moisture content in the mixed solution from increasing too high. This is because if the moisture content in the mixed solution is increased, a reaction between fibrinogen and thrombin occurs before attachment, thus affecting the attachment operation. Furthermore, in order to obtain an excellent adhesive force after transplantation, it is preferable that the fibrinogen and the thrombin are attached onto amniotic membrane in a state in which they are not affected by each other in advance. Taken into consideration the above-mentioned reasons, it is preferable that the solvent of fibrinogen and the solvent of thrombin respectively employ a solvent having water solubility with less moisture content and having volatility.

The application, dropping and the like of the fibrinogen solution and the thrombin solution or the mixed solution of fibrinogen and thrombin are carried out typically on the entire surface of the amniotic membrane. However, the application, dropping and the like may be carried out only in a certain region (for example, a plurality of regions aligned in a spot-like manner with a certain interval in between, or only in a peripheral portion), or may be carried out while varying the attached amounts.

In the above-mentioned method, fibrinogen and thrombin are attached at the same time. However, each component may be attached in separate processes. That is to say, the attachment of fibrinogen and the attachment of thrombin may be carried out as two steps. However, it is preferable that fibrinogen and thrombin are attached in one step by using a mixed solution of fibrinogen and thrombin because the operation can be simplified and fibrinogen and thrombin can be attached in a more uniform dispersion state.
Note here that fibrinogen and thrombin can be prepared from the blood by a routine method. The recombinant fibrinogen and the recombinant thrombin can be used. In this case, fibrinogen and thrombin can be prepared from a culture solution or a cell homogenized solution of an appropriate cell culture by a routine method. Furthermore, commercially available fibrinogen and thrombin may be used. For example, human-derived fibrinogen is available from Baxter. Similarly, human-derived thrombin is available from Baxter.

In addition to fibrinogen and thrombin, aprotinin may be attached to amniotic membrane. That is to say, in this embodiment, a step of attaching aprotinin (step b-1) is further carried out. The attachment of aprotinin can be carried out by the same means and procedure as those of fibrinogen, and the like. By application, dripping, dipping, spraying, immersing, and the like, of an aprotinin solution, aprotinin can be attached to the surface of amniotic membrane. The aprotinin solution can be prepared by dissolving aprotinin into a sodium chloride solution (for example, 0.85% solution), a potassium chloride solution, a calcium chloride solution, a magnesium chloride solution, and the like.
Note here that aprotinin can be prepared from bovine pancreas according to a routine method. Recombinant aprotinin may be used. In this case, aprotinin can be prepared from a culture solution or a cell homogenized solution of an appropriate cell culture by a routine method. Commercially available aprotinin may be used. For example, bovine-derived aprotinin is available from Bayer.
A step of attaching aprotinin can be carried out singly but it is preferable that it is carried out simultaneously with a step of attaching fibrinogen and thrombin. This is advantageous because an operation of attaching adhesive components is simplified as a whole. Furthermore, this is advantageous because fibrinogen, thrombin and aprotinin can be attached to the surface of amniotic membrane in a state in which they are dispersed more uniformly. For example, a mixed solution of fibrinogen, thrombin and aprotinin is prepared and this mixed solution is applied. Thereby, the above-mentioned three components can be simultaneously attached to amniotic membrane. The order of mixing these three components are not particularly limited.

The attachment of fibrinogen and thrombin may be carried out to one side of the surface or both sides of the surface of amniotic membrane. In the former case, in principle, regardless of the presence or absence of the epithelium of amniotic membrane, fibrinogen and thrombin are attached to the side (that is, the side of the chorionic membrane) opposite to the epithelium side (the epithelium has been present).

After fibrinogen and thrombin (further, aprotinin in some cases) are attached, drying process is carried out if necessary. Thus, a sheet-shaped composition having an excellent preservation stability can be obtained. Furthermore, the thus obtained sheet-shaped composition is preferable from the viewpoint of handling (transport, transplantation operation).
The drying process to be employed may be a usual drying process method such as air drying, vacuum drying, drying under reduced pressure, and lyophilization.

In one embodiment of the present invention, a cell layer using cells of biological origin is formed on amniotic membrane. This cell layer is formed before fibrinogen and thrombin are attached to the surface of amniotic membrane. That is to say, in this embodiment, after a cell layer is formed on amniotic membrane, adhesive components such as thrombin are attached to the surface of amniotic membrane (surface on which a cell layer is not formed).
The step of forming a cell layer is carried out by the following procedure. Firstly, an appropriate cell of biological origin is prepared (step of preparing a cell of biological origin). As the cell of biological origin, a cell applicable to the use of finally obtained biological tissue sheet is used. For example, in a case where a sheet for regeneration of the skin epidermal tissue is intended to be produced, epidermal cells (or stem cells or precursor cells thereof) and hair follicle epithelial cells (or stem cells or precursor cells thereof) are preferably used. Similarly, for the purpose of regenerating a cornea epithelial tissue, corneal epithelial cells (or stem cells or precursor cells thereof) are preferably used, and for the purpose of regenerating a mucosal epithelial tissue, mucosa epithelial cells (or stem cells or precursor cells thereof) are preferably used. Examples of the mucosa epithelial cell include an oral mucosal epithelial cell, an intestinal tract mucosa epithelial cell, a respiratory tract mucosa epithelial cell.
A method of preparing cells of biological origin is described taken a skin epidermal cell, a corneal epithelial cell, an oral mucosal epithelial cell, an intestinal tract mucosa epithelial cell, and a respiratory tract mucosa epithelial cell as examples.

### (Skin Epidermal Cell)

Firstly, when the skin is collected, a site to be collected is disinfected with disinfectant such as povidone iodine prophylactically in advance and antifungal agent is externally applied thereto, followed by collecting a small skin piece in accordance with skin biopsy. In culturing epidermal keratinocytes, fatty tissue and dermis are removed from the skin piece as much as possible by using scissors and washed with Dulbecco's phosphate buffer (PBS) several times and soaked in 70% ethanol for one minute for sterilization. The piece is cut into a strip shape, soaked in Dispase solution and stood still over night at 4°C. Then, epidermis is peeled off from the dermis. The peeled epidermis is washed, followed by disentangling the epidermal piece so as to prepare suspending solution of epidermal keratinocyte. The cells are suspended in a serum free culture medium and seeded on a collagen-coated dish, and subculture is carried out.

### (Corneal Epithelial Cell)

Corneal epithelial cells can be obtained from a corneal limbus tissue. For example, endothelial cells are peeled off and removed from corneal limbus tissue, and conjunctiva is excised so as to form a single cell suspension. Then, this is preserved in a nitrogen tank, and then rapidly melted at 37°C so as to adjust a corneal epithelial cell suspending solution. If necessary, subculture is carried out. For subculture, for example, EpiLife^{™} (Cascade), an MCDB153 medium (NISSUI PHARMACEUTICAL CO., LTD.), which are serum free media, and media produced by modifying the amino acid composition, etc. of the above-mentioned media can be used.

### (Oral mucosal epithelial cell)

As the oral mucosal epithelial cells, cells existing in the dental root part (oral crevicular mucosal epithelial cells), cells of labial part, cells of palate part, cells of buccal part, and the like, can be used. Among them, it is particularly preferable to use oral crevicular mucosal epithelial cells because of the high proliferation ability and low antigenicity. The oral mucosal epithelial cells can be collected by ablating a site where targeted cells exist with the use of a scalpel, or by scraping it out. Oral crevicular mucosal epithelial cells can be collected by separating oral mucosal epithelial cells from the enamel cement transition portion and collecting the cells from the obtained tissue piece. Note here that in order to remove impurities such as connective tissue, preferably, a treatment with enzyme such as dispase or trypsin, etc., filtration treatment are carried out.

### (Intestinal tract mucosa epithelial cell)

The intestinal tract mucosa epithelial cells are collected from intestinal tract epithelium tissue through an endoscope of the large intestine, or by usual technique at the time of abdominal section. Furthermore, epithelial cells can be removed from tissue by laser capture microdissection. The technique of the present invention can be applied to a biological tissue sheet produced by using epithelial cells from all the human digestive tract such as esophagus, upper stomach, duodenum, small intestine, and large intestine. When ulcer, inflammation, or the like, causes injuries of human digestive tract epithelium, cells derived from bone marrow play a roll as a rescue with respect to emergency, so that the epithelium is repaired. The digestive tract epithelial cells, although a part of them, are also made from bone marrow. In this sense, the present invention can be regarded to have significance that is equivalent to that using corneal epithelial cells. In general, an epithelial cell made of bone marrow, which is usually only several cells per 1000 cells, are increased 50 to 100 times in the process in which ulcers (wounds) on the internal surface of the digestive tract, which are generated by, for example, gastric ulcer and colitis, are being cured. It is determined that about 1 of 10 digestive tract epithelial cells are derived from the bone marrow. The biological tissue sheet derived from the digestive tract mucosa epithelial cells are extremely significant because they urge the regeneration of intestinal tract epithelium with respect to ulcer and inflammation of intestine diseases which are designated intractable diseases, that is, severe intestinal tract infectious diseases such as ulcerous colitis, Crohn's disease, Behchet's disease, and the like. The effectiveness with respect to intestinal tract allergy can be expected.

### (Respiratory Tract Mucosa Epithelial Cell)

Respiratory tract mucosa epithelial cells can be easily obtained from biopsy tissue of the respiratory tract mucosa. Similar to the above-mentioned tissue, in order to remove impurities such as connective tissue, it is preferable that treatment with enzyme such as Dispase, trypsin, and the like, or filter treatment is carried out. The respiratory tract mucosa epithelial cells play an important role for pathologic conditions of various infectious diseases via biosyntheses and release of β defensin. Furthermore, respiratory tract mucosal epithelium also plays an important role in asthma or allergic disease. Providing biological tissue sheet produced by the respiratory tract mucosa epithelial cells according to the present invention to the respiratory tract mucosa having tissue disorder would lead to not only carrying out emergency treatment but also providing artificial respiratory tract. In particular, immunosuppression effect of the sheet with amniotic membrane is useful.

It is preferable that after tissue is collected, oral mucosal epithelial cells, intestinal tract mucosa epithelial cells, and the like, are subjected to a treatment with enzyme such as Dispase, trypsin, and the like, or filter treatment in order to remove impurities such as connective tissue.

It is preferable that the cells of biological origin are prepared from a person (recipient) who undergoes transplantation. That is to say, it is preferable that a donor of cells of biological origin is identical to a recipient of the biological transplantation sheet. By using such autologous cells, problem as to immunological rejection is avoided.

The prepared cells of biological origin are seeded onto amniotic membrane (step of seeding cells of biological origin onto amniotic membrane), followed by culturing thereof (step of culturing and proliferating the seeded cells of biological origin).
In this embodiment, it is particularly preferable to use amniotic membrane from which the epithelium has been removed. By removing the epithelium, the reduction of antigenicity can be expected. Furthermore, since unnecessary cells are removed in advance, target cell layers can be formed excellently. When amniotic membrane from which the epithelium has been removed is used, it is preferable that cells of biological origin are seeded on the side of the exposed surface from which the epithelium has been removed (that is to say, side of the basal membrane). It is thought that this side of the surface is rich in type IV collagen, so that the proliferation and stratification of the seeded cells of biological origin can proceed excellently.

Herein, by using two types of cells, a hybridized cell layer may be formed. A method of forming a cell layer in such a case is described in detail hereinafter, taken the case where a sheet-shaped composition for reconstructing the corneal epithelium as an example.
Firstly, one of the cell types used for forming a cell layer (the first cells), cells derived from mucosal epithelium such as oral mucosal epithelium, conjunctival epithelium, and nasal mucosal epithelium, or undifferentiated cells capable of constructing such mucosal epithelium can be preferably used. On the other hand, as the cell type (the second cells) used for forming a cell layer together with the first cells, corneal epithelial cells, conjunctival epithelial cells, or amniotic membrane epithelial cells can be preferably used. These cells can be collected from a living tissue in which these cells are present. Specifically, for example, a part of the tissue in which target cells exist is collected by using a surgical knife and the like, through the treatment such as removing of the connective tissue, separation of cells, and the like, and formed in a state of the cell suspending solution (suspension). Note here that as the first cells, two or more different types of cells may be used. Similarly, as the second cells, two or more different types of cells may be used.

It is suggested that oral mucosal epithelium that is suitable for a collection source of the first cells includes stem cells. Therefore, it is thought that the oral mucosal epithelium can easily carry out differentiation induction for forming cells capable of forming an epithelium-like cell layer. Furthermore, the use of oral mucosal epithelial cells has advantages that they are collected easily, a large number cells can be collected, and furthermore, even in the case of treating corneal disease occurring in bilateral eyes, autologous cells can be used so as to prepare a transplantation material, and the like. In particular, also for patients from whom corneal epithelial cells cannot be collected, transplantation material derived from the autologous cells can be provided. This advantage is expected to radically dissolve the problem of clinically important rejection.
As the oral mucosal epithelial cells, cells existing in the dental root part (oral crevicular mucosal epithelial cells), cells of labial part, cells of palate part, cells of buccal part, and the like, can be used. Among them, it is particularly preferable to use oral crevicular mucosal epithelial cells because of the high proliferation ability and low antigenicity. The oral mucosal epithelial cells can be collected by ablating a site where target cells exist with the use of a scalpel, or by scraping it out. Oral crevicular mucosal epithelial cells can be collected by separating the oral mucosal epithelium that is attached to an extracted tooth from the enamel cement transition portion and collecting the cells from the oral mucosal epithelium. Note here that in order to remove impurities such as connective tissue, preferably, a treatment with enzyme such as dispase or trypsin, etc., filtration treatment are carried out.
Oral mucosal epithelial cells collected from a person other than the patient who is intended to undergo a transplantation of the sheet-shaped composition of the present invention can be used. However, taken immunorejection into consideration, it is preferable that oral mucosal epithelial cells are collected from the oral cavity of the patient and used for culture.
The oral mucosa has high proliferation potency. In the oral mucosa, generally, since the injury is cured after the operation by administering internal antimicrobial drug and carrying out disinfection with Isodine, and the like, for several days, the invasion with respect to the patient who was subjected to collection of mucosa seems to be light.

On the other hand, as the second cells, another individual's (allo) corneal epithelial cells can be preferably used. As such corneal epithelial cells, cells from donor's eyeball free from infection are available from, for example, eye bank (Northwest eye bank, etc.). The cells that can be used as the second cell are not limited to the corneal epithelial cells. Conjunctival epithelial cells, amniotic membrane epithelial cell, and the like, may be used. However, when the corneal epithelial cells constituting the corneal epithelium in a living organism or the conjunctival epithelial cells existing in the vicinity thereof are employed, it is thought that a sheet-shaped composition capable of reproducing the property of the corneal epithelium more excellently. As a result of the present inventor's investigation, when the corneal epithelial cells are used as the second cell, it was confirmed that a cell layer similar to the corneal epithelium was constructed. This fact supports the above-mentioned prediction and supports that the corneal epithelial cells are particularly preferable for the second cells. On the other hand, it was confirmed that when the amniotic membrane epithelial cells were used as the second cell, a cell layer capable of excellently reproducing the properties required for the cornea was formed. This fact shows that the amniotic membrane epithelial cells can be also preferably used as the second cells.

Autologous cells can be used as the second cells. However, when other individuals' cells are used, the cells can be obtained more easily. For example, even when a sheet-shaped composition for the treatment of a patient with bilateral eye disease is produced, the corneal epithelial cells as the second cells are available.

The separately prepared first cells and the second cells (hereinafter, also referred to as "the first cells, and the like") are seeded on amniotic membrane and cultured. In general, the first cells and the second cells, which are prepared in a form of a cell suspending solution, are dripped on amniotic membrane and cultured.
Typically, the seeding of the first cells and the seeding of the second cells are carried out simultaneously (herein, "simultaneously" includes not only a case where the seeding is carried out literally simultaneously but also a case where the first seeding is carried out and then the second seeding is carried out without substantial time interval). The first and second cells may be seeded at different timing. For example, the second cells may be seeded several minutes to several hours after the first cells are seeded. Thus, by shifting the time of seeding cells, for example, a cell layer in which a region rich in cells derived from the first cell is localized can be constructed. Thereby, the structure of the cell layer and the property thereof can be changed or adjusted.

The ratio of the first cells and the second cells to be seeded is not particularly limited. Typically, substantially the same number of the first and second cells are seeded. In an experiment in which the oral mucosal epithelial cells were used as the first cells and the corneal epithelial cells were used as the second cells, the ratio of the number of the first cells: second cells were changed to 3 : 7, 5 : 5, and 7 : 3 and comparison was carried out. As a result, no difference in terms of the cell proliferation and stratification were clearly observed among them (data not shown).

When the first and second cells are cultured on amniotic membrane, these cells are proliferated and a cell layer is formed (in this process, at least a part of the cells are thought to be differentiated). After the formation of a cell layer, a step of bringing the surface layer of the cell layer into contact with the air is carried out. This step is also referred to as air lifting in this specification. This step is carried out for differentiation of cells forming a cell layer and inducing the barrier function.
This step can be carried out by lowering the surface of the culture medium by temporarily removing a part of the culture medium by using a dropper, a pipette, and the like, thereby temporarily exposing the outermost layer of the cell layer to the outside of the culture medium. Alternatively, this step can be carried out by lifting up the cell layer together with the amniotic membrane, thereby temporarily exposing the outermost layer from the culture medium surface. Furthermore, by using the tube etc., the air may be fed into the culture medium so as to bring the uppermost layer of the cell layer into contact with the air. From the viewpoint of the ease in operation, it is preferable that by lowering the surface of the culture medium, thereby exposing the outermost layer of the cell layer to the outside.
The duration for carrying out this step, that is, the period of time when the uppermost layer of the cell layer is brought into contact with the air differs depending upon the state of the cells, culture conditions, and the like, but the duration may be, for example, three days to two weeks, preferably within a week, and further preferably within three days.
According to the above-mentioned method of the present invention, on the amniotic membrane, a corneal epithelium-like cell layer, in which the first cells and the second cells are stratified, is formed. The thus obtained sheet-shaped composition together with the amniotic membrane used as a substrate of the first cells and the second cells can be used as a transplantation material (substitute for the corneal epithelium) for patients with injured or defective cornea. In this case, the sheet-shaped composition is transplanted to the corneal epithelium defective part so that the amniotic membrane is located to the side of the eyeball.

In one embodiment of the present invention, cells of biological origin are cultured in the presence of support cells. The support cell is also referred to as a feeder cell and supplies a culture medium with a growth factor, etc. When the cells of biological origin are cultured in the coexistence of the support cells, the proliferation efficiency of cells is improved. As the support cell, for example, a 3T3 cell (Swiss mouse 3T3 cell, mouse NIH3T3 cell, 3T3J2 cell, etc.) and the like, may be used. Among them, it is preferable to use a mouse NIH3T3 cell as a support cell from the viewpoint of proliferation efficiency, ease in handling, etc.
It is preferable that the support cells are inactivated by using mitomycin C, etc. This is advantageous because the inhibition of the proliferation of the cells of biological origin due to the proliferation of the support cells themselves is prevented, and the proliferation efficiency of the cells of biological origin is enhanced. Such inactivation can be carried out by a radiation treatment, and the like.

The cell density of the support cells may be, for example, about 1×10² cells/cm² or more, preferably in the range from about 1×10² cells/cm² to about 1×10⁷ cells/cm², and further preferably in the range from about 1×10³ cells/cm² to about 1×10⁵ cells/cm². As to the ratio with respect to the number of the first cells and the second cells, culture may be carried out under the conditions in which the number of the support cells to be used may be, for example, 1/10³ times to 1×10² times, and preferably 1/10² times to 1 time as the total number of cells or biological origin. When the number of the support cells is small, the proliferation rate of the first cells and the like is lowered; and when it is too small, excellent proliferation and stratification of cells of biological origin cannot be obtained. On the other hand, it is not preferable that the number of the support cells is too large, because the proliferation rate of the oral mucosal epithelial cells is lowered.

When the cells of biological origin are cultured in the coexistence of support cells, it is preferable that an isolation membrane having a pore size through which the support cells cannot pass is provided between the support cells and the amniotic membrane. The use of the isolation membrane makes it possible to prevent the support cells from entering the side of the amniotic membrane (i.e. the side of living organism cells) at the time of culturing. As a result, the support cells may not be mixed in the finally obtained sheet-shaped composition. This means that a sheet-shaped composition being free from problem of immunological rejection by the support cells can be constructed. Clinically, this is extremely significant.
As the isolation membrane, an isolation membrane having a pore size through which the support cells cannot pass can be used by appropriately selecting the known membrane. For example, a polycarbonate membrane having a pore size of about 0.4 µm to 3.0 µm can be used. A material of the isolation membrane is not particularly limited. In addition to polycarbonate, polyester and the like may be used. Such isolation membranes are on the market and easily available.
Example of the culture method using an isolation membrane include the following method. Firstly, inactivated support cells are seeded and cultured on a container such as a dish (a first container), thereby forming a layer of support cells on the surface of the container. Next, a second container, which has a bottom face made of an isolation membrane, is set in the first container so that the bottom face of the second container is located in a culture medium. Then, the amniotic membrane is formed on the bottom face, that is, on the isolation membrane. Then, on the collagen layer, the cells of biological origin are seeded and cultured.
In one example, on bottom surface of the second container, amniotic membrane is previously formed (for example, on the bottom surface of the second container, the amniotic membrane from which an epithelium has been removed is placed. In this state, drying process is carried out). This second container is set in the first container in which support cells are seeded, and then on the collagen layer, the first cells and the like may be seeded and cultured.

The culture medium used for culturing the cells of biological origin is not particularly limited as long as the cells can be proliferated and stratified. For example, a culture medium, in which DMEM (Dulbecco's modified Eagle's medium) that is generally used for growing epithelial cells and Ham's F12 medium are mixed with each other at the predetermined ratio, and FBS, growth factor, antibiotics, and the like are added, may be used. Specific examples include a mixed culture medium of DMEM and Ham's F12 medium (mixing volume ratio of 1 : 1) to which FBS (10%), insulin (5 mg/ml), cholera toxin (0.1 nM), epithelial cell growth factor (EGF) (10 ng/ml) and penicillin-streptomycin (50 IU/ml) are added. Furthermore, a mixed culture medium of DMEM and Ham's F12 medium to which triiodothyronine (for example, 2 nM), glutamine (for example, 4 mM), transferrin (for example, 5 mg/ml), adenine (for example, 0.18 mM), and/or hydrocortisone (for example, 0.4 mg/ml) are further added, may be used.

The cells of biological origin may be cultured in the absence of xenogeneic cells. The "the absence of xenogeneic cells" in the present invention means that cells of animals different from the cells of biological origin are not used as a condition for culturing the cells of biological origin. Specifically, when human cells (for example, human skin epidermal cells or human corneal epithelial cells) are used, the condition means that cells from the animal species other than human, for example, a mouse, a rat, or the like, are not present (do not coexist). When cells are cultured in such a condition, xenogeneic components (including xenogeneic cells themselves) may not be contaminated in the finally obtained transplantation material (that is, sheet-shaped composition).
The culture medium used for culturing cells of biological origin is not particularly limited as long as it allows the cells to proliferate. For example, an MCDB153 medium (NISSUI PHARMACEUTICAL CO., LTD.), EpiLife^{™} (Cascade), and media produced by modifying the amino acid composition, etc. of these media, a culture medium mixing DMEM (Dulbecco's modified Eagle's medium) and Ham's F12 medium, which are usually used for growing epithelial cells, at a predetermined ratio can be used. In particular, in the present invention, it is preferable that a culture medium that does not contain serum and xenogeneic proteins is used. On the other hand, a culture medium containing growth factor, antibiotics, and the like may be used. However, it is preferable to use a culture medium that does not contain any serum. That is to say, it is preferable that serum free culture is employed as a culture method in the present invention. This is advantageous because problem such as immunological rejection due to the contamination of components derived from the serum can be avoided. Note here that culture may be carried out in a culture medium containing serum, in this case, however, it is preferable to use allogeneic serum (when cells of human origin is used, serum of human origin) or to use autologous serum. Needless to say, if possible, it is preferable to use autologous serum capable of avoiding causing the immunorejection.
The culture conditions may be changed in the course of culture for the purpose of excellently proliferating cells of biological origin.

As a result of the culturing step, cells of biological origin proliferate on the amniotic membrane. When the surface layer of the thus obtained cell layer is required to be keratinized (for example, a case where epidermal cells are used so as to form a skin epidermal sheet or a case where corneal epithelial cells are used so as to form a corneal epithelial sheet), the above-mentioned Air-lifting may be carried out.

The cells of biological origin are seeded on the amniotic membrane so that, for example, the cell density becomes about 1×10³ cells/cm² or more, preferably in the range from about 1 × 10³ cells/cm² to about 1×10⁷ cells/cm², and further preferably in the range from about 1×10⁴ cells/cm² to about 1×10⁶ cells/cm².

In one preferable embodiment, amniotic membrane is placed on a collagen matrix containing human fibroblasts, which has been previously prepared, and then the cells of biological origin are seeded on the amniotic membrane and cultured. That is to say, in this embodiment, a step of culturing human fibroblasts in a collagen gel (the step B) and a step of placing amniotic membrane on the collagen gel, followed by seeding or placing cells of biological origin on the amniotic membrane (the step C) are carried out. The sheet-shaped composition that has been produced by this procedure has come to contain the cells of biological origin proliferated on the amniotic membrane placed on the collagen gel containing human fibroblasts. The sheet-shaped composition of this embodiment can be also used as a transplantation material after the collagen matrix is removed. Alternatively, the sheet-shaped composition of this embodiment can be also used as a transplantation material in a state in which it includes the collagen matrix.
"Collagen gel" functions as a culture substrate of human fibroblasts. The types of collagens as a material of the collagen gel are not particularly limited, and type I collagen, type III collagen, and type IV collagen, and the like, can be used. A plurality of collagens can be used in combination thereof. Such collagens can be extracted and purified from the connective tissue of the skin and cartilage, etc. of animals such as pig, bovine, sheep, etc., by an acid solubilization method, an alkali solubilization method, and an oxygen solubilization method, and the like. For the purpose of deteriorating the antigenicity, it is preferable to use so-called atherocollagen obtained by removing telopeptide by a treatment with the use of catabolic enzyme such as pepsin, trypsin, etc. As materials of the collagen gel, a collagen derived from amniotic membrane, particularly derived from human amniotic membrane may be used. Herein, the collagen layer is "derived from amniotic membrane" means that the collagen gel is obtained by using amniotic membrane as a starting material.

The origin of the human fibroblasts contained in the collagen gel is not particularly limited and it may be derived from any tissue as long as the tissue produces collagen. Human fibroblasts prepared from, for example, skin tissue, oral mucosa tissue, and the like, can be used.

A specific example of the method of producing a collagen matrix is shown. Firstly, human fibroblasts are prepared by the following procedure. The skin is collected, and then dermis is peeled off from the skin. The dermis is cut in strips and is brought into close contact with a dish coated with type I collagen. After static culture, human fibroblasts migrated from the dermis strip are subcultured. Cells are peeled off from the bottom surface of the dish and a cell suspending solution is prepared. The cell suspending solution is seeded on a cell culture dish. Appropriately, cells are cryopreserved (for example, stored in liquid nitrogen).
Meanwhile, a neutralized collagen solution is prepared by using type I collagen (see the below-mentioned Example). This is added in a culture container (for example, a culture insert) and stood still for ten minutes at room temperature so as to be gelled. Next, human fibroblasts in a logarithmic growth phase, which has been cultured by the above-mentioned method in advance, are mixed with this gel and gelled again. Thereafter, static culture is carried out. A collagen matrix containing human fibroblasts can be obtained by the above-mentioned procedure. This inventiveness allows the collagen matrix to have necessary strength and to have amniotic membrane layer or cells of biological origin to be mounted thereon, which makes a base of the present invention. A separately prepared amniotic membrane can be placed on (brought into contact with) the collagen matrix. Thereafter, cells are seeded and cultured in accordance with the above-mentioned procedure.

Hereinafter, the present invention is described with reference to Examples (including Experiment examples).

### [Examples]

### 1. Production of adhesive attached sheet

### 1-1. Preparation of mixed solution of adhesive components

Firstly, 84 mg of fibrinogen (Baxter) and 700 µl of 94% ethanol were mixed, agitated by a homogenizer, and stood still at 4°C for 20 hours (solution A). On the other hand, 2 mg of thrombin (Baxter) and 35 µl of 100% ethanol were mixed, agitated by pipetting, and stood still at -30°C for 16 hours (solution B).
The solution A and the solution B were mixed, and then 7 µl of aprotinin (Baxter), which had been adjusted to 3000 KIU/ml, was added thereto. The thus obtained mixed solution containing three components was subjected to the below-mentioned attaching treatment.

### 1-2 Production of adhesive components attached-amniotic membrane sheet with epithelium

A sheet in which adhesive components were attached to amniotic membrane (containing the epithelium) (hereinafter, the sheet is referred to as "adhesive components attached-amniotic membrane sheet with epithelium") was prepared by the following procedure.

### 1-2-1 Collection of amniotic membrane

After giving a pregnant woman who does not have a systemic complication and would undergo Caesarean section sufficient informed consent together with an obstetrician in advance, the amniotic membrane was obtained during the Caesarean section in the operation room. The operation was carried out cleanly. In accordance with the operation work, the operators washed hands, and then wore a special gown. Before delivery, a clean vat for obtaining the amniotic membrane and physiologic saline for washing were prepared. After delivery, the placenta tissue was transferred to the vat and the amniotic membrane tissue was manually removed from the placenta. A portion where the amniotic membrane and the placenta were strongly adhered to each other was separated with scissors.

### 1-2-2 Treatment of amniotic membrane

Treatment process of amniotic membrane included: (1) washing, (2) trimming, and (3) storing sequentially in this order. Throughout the entire processe, operation is desired to be carried out in a clean draft. For all containers and instruments for use, those sterilized were used, and for dishes, etc. sterilized disposable ones were used. The obtained amniotic membrane was washed for removing blood component attached thereto and further washed in a sufficient amount of physiological saline (0.005% ofloxacin was added). Then, the amniotic membrane was transferred to a phosphate buffer solution (PBS) in a dish and cut and divided into the size of about 4 × 3 cm with scissors. The divided pieces of amniotic membrane were stored in several dishes filled with a stock solution, and thereafter amniotic membranes in good condition were selected among them.

### 1-2-3 Storage of amniotic membrane

One cc each of stock solution was placed in 2 cc sterilized cryotube and one sheet each of the amniotic membrane, which had been obtained, washed and selected, was placed and labeled, then stored in a refrigerator at -80°C. For the stock solution, 50% sterilized glycerol in DMEM (Dulbecco's Modified Eagle Medium: GIBCOBRL) was used. The expiration date for use of stored the amniotic membrane was set to be at three months and expired amniotic membrane was disposed of by incineration.

### 1-2-4 Drying process

The stored amniotic membrane was sandwiched by using one set of sterilized plastic frames and then fixed with a clip. The amniotic membrane together with the frame were transferred to a deep freezer (-80°C). After it was confirmed that the amniotic membrane was frozen, lyophilization treatment (-110°C, about one hour) was carried out by using a vacuum lyophilizer (Yamato, NEOCOOL). According to the instruction for use, conditions were set so as to obtain a sufficient dried product.

### 1-2-5 Application of adhesive components onto dried amniotic membrane sheet

The solutions A and B that were prepared in 1-1 and an aprotinin solution were mixed and sufficient pipetting was carried out (mixed solution). Next, this mixed solution was dripped on the surface (at the side of chorionic membrane) of the dried amniotic membrane that was produced in 1-2-4 so that the mixed solution was spread to the entire surface (attached amount of fibrinogen: 5.5 mg/cm², attached amount of thrombin: 0.13 mg (1.4 U)/cm² and attached amount of aprotinin: 1.4 KIU/cm²), followed by drying under reduced pressure at ordinary temperature for two hours. Subsequently, dried amniotic membrane was taken off from the frame, transferred into a two-layer structure bag made of polyamide nylon at the outside and polyethylene at the inside, and vacuum-packed by using a home vacuum packer (Flaem Nouva, MAGIC VAC). The thus obtained amniotic membrane that is in vacuum-packed was irradiated with y ray (about 25 kGy) so as to be sterilized. The sterilized amniotic membrane was stored at ordinary temperature in a state in which it was vacuum-packed right before use (adhesive components attached-amniotic membrane sheet with epithelium).

### 1-3 Production of adhesive components attached-amniotic membrane sheet without epithelium

A sheet in which adhesive components were attached to amniotic membrane from which the epithelium had been removed (hereinafter, referred to as "adhesive components attached-amniotic membrane sheet without epithelium") was prepared by the following procedure.

### 1-3-1 Removing of epithelium from amniotic membrane

The amniotic membrane, which had been collected by the above-mentioned procedures (1-2-1 to 1-2-3) and stored, was thawed at room temperature, and then sufficiently washed with a sterilized phosphate buffer solution (PBS) in a dish. After washing, the amniotic membrane was stored in a 0.02°,% EDTA solution (Nacalai tesque) at 37°C for 2 hours, and then the epithelium was mechanically scraped off by using a cell scraper (Nunc, USA). The thus obtained amniotic membrane without epithelium was subjected to drying process in accordance with the above-mentioned procedure (1-2-4 and 1-2-5), and application of adhesive components (attached amount of fibrinogen: 0.5 mg/cm², attached amount of thrombin: 12 µg/cm², attached amount of aprotinin: 0.12 KIU/cm²), and then vacuum-packed adhesive components attached-amniotic membrane sheet without epithelium was obtained.

### 1-4 Production of adhesive components attached-amniotic membrane sheet with cell layer (1) (adhesive components attached-cultured corneal sheet).

A sheet in which a cell layer derived from corneal epithelial cells was formed on amniotic membrane (without the epithelium) and further adhesive components were attached to the surface of the amniotic membrane (hereinafter, also referred to as "adhesive components attached- cultured corneal sheet") was prepared by the following procedure.

### 1-4-1 Removing of epithelium from amniotic membrane

The amniotic membrane, which had been collected by the above-mentioned procedures (1-2-1 to 1-2-3) and stored, was thawed at room temperature, followed by sufficiently washing with a sterilized phosphate buffer solution (PBS) in a dish. After washing, the amniotic membrane was stored in a 0.02% EDTA solution (Nacalai tesque) at 37°C for 2 hours, and then the epithelium was mechanically scraped off by using a cell scraper (Nunc, USA). The thus obtained amniotic membrane without containing the epithelium was used as a substrate for the following cell culture.

### 1-4-2 Collection of corneal epithelial cells

A corneal limbus tissue piece (about 5 mm × 10 mm) was collected from the corneal limbus of a 6-week old Japanese white rabbit by using a surgical knife. The collected tissue piece was immersed twice in a phosphate buffer solution (PBS) containing 50 IU/ml penicillin streptomycin and Gentacin for 30 minutes under the condition of room temperature. Thereafter, the tissue was immersed in a phosphate buffer solution (PBS) containing 1.2U Dispase (Nacalai tesque) for one hour at 37°C and then immersed in 0.05% trypsin-EDTA solution (GBCOBRL) for 15 minutes so as to separate cells. An enzyme activity was stopped by immersing the tissue in DMEM containing 10% fetal bovine serum (FBS). Thereafter, excess tissues were removed by using a 60 µm cell-filter so as to isolate the corneal epithelial cells (corneal epithelial cell suspension).

### 1-4-3 Preparation of co-cultured cell

As the co-culture cells (support cells), NIH-3T3 cells (hereinafter, referred to as "3T3 cells") were used. The 3T3 cells that had been cultured in advance and become confluent in 75F flask (BD product of Falcon) were immersed in 0.05% mitomycin C solution for two hours so as to suppress the proliferation activity. Sequentially, they were washed with a phosphate buffer solution (PBS) several times so as to remove mitomycin C, followed by treating with 0.05% trypsin-EDTA solution. Thus, a suspension of 3T3 cells was prepared.

### 1-4-4 Cell culture and induction of mucosal epithelium

By using human amniotic membrane, which had been prepared in 1-4-1 and from which the epithelium had been scraped, as a substrate, the corneal epithelial cells were co-cultured with 3T3 cells that had been subjected to the above-mentioned treatment by the following procedure. As culture instruments, a 6-well culture dish (Coming, NY) and a culture insert (a container into which a culture is inserted) (made of polycarbonate, average pore size: 3.0 µm, Coming NY) were used.
First of all, 3T3 cell suspension was seeded on the culture dish so that the cell density was about 1×10⁴ cells/cm² and cultured under conditions at 37°C and in 5% CO₂. Furthermore, the amniotic membrane substrate was allowed to stand still so as to be attached on the culture insert with the side from which the epithelium had been scraped upward, and dried for 10 minutes at room temperature. Thereafter, on the culture insert to which the amniotic membrane was attached, corneal epithelial cell suspending solution was seeded so that the cell density became about 1 × 10⁴ cells/cm².
After the above-mentioned operation, as shown in Fig. 1, the culture insert was disposed in the culture dish and 3T3 cells and corneal epithelial cells were cultured in the same culture medium. Note here that Fig. 1 is a schematic cross-sectional view showing a state during culture. In the culture dish 1, the culture insert 2 is placed and on the bottom surface of the culture dish 1, the 3T3 cell layer 5 is formed. Furthermore, on the bottom surface of the culture insert 2, the amniotic membrane 3 is placed, and the corneal epithelial cells 4 are cultured on the amniotic membrane 3. Reference numeral 6 denotes a culture medium.
As the culture medium, a DMEM / Ham's F12 mixture medium (mixing volume ratio: 1:1) containing 10% FBS, insulin (5 mg/ml), cholera toxin (0.1 nM), penicillin-streptomycin (50 IU/ml) and human recombinant epithelial cell growing factor (EGF) (10 ng/ml) was used.
After the culture for seven days (submerge), another culture for about three days by a so-called Air-lifting method was carried out to induce differentiation of the mucosal epithelium. The Air-lifting method is a method of lowering the level of the culture medium to the level of the surface of the cell layer formed on the amniotic membrane to bring the surface of the cell layer into contact with the air. During submerging, the culture medium was replaced with new one every other day. After air-lifting, the culture medium was replaced with new one every day.
By culturing the above-mentioned method, a cell layer in which 5 to 6 layers are stratified for about 10 days (including three days of culture by the air-lifting method) was formed (cultured corneal sheet).

### 1-4-5 Attachment of adhesive components

After the cultured corneal sheet that had been obtained as mentioned above was taken out from the culture solution, one side of the surface (at the side of chorionic membrane) which is opposite to the side of the surface on which the cell layer was formed was subjected to air drying by blowing air so as to be in a semidry state. Next, the surface of the semidry amniotic membrane was immersed in a mixed solution including fibrinogen, thrombin and aprotinin (prepared by the procedure similar to the procedure described in 1-2-5), followed by air drying so as to be in a semidry state. During this series of operation, the side of the surface on which cells were cultured was maintained in a wet state. As a result of the above-mentioned operation, a sheet-shaped composition (adhesive attached-cultured corneal sheet) was obtained. In the sheet-shaped composition, adhesive components are attached on one side of the surface of the amniotic membrane, which is opposite to the side on which a cell layer was formed.

### 1-5 Production of adhesive components attached-amniotic membrane sheet with cell layer (2) (adhesive components attached-cultured corneal sheet)

A sheet in which a cell layer derived from epidermal keratinocytes was formed on amniotic membrane (from which epithelium had been removed) and further adhesive components were attached to the surface of the amniotic membrane (hereinafter, also referred to as "adhesive components attached-cultured corneal sheet") was prepared by the following procedure.

### 1-5-1 Removing of epithelium from amniotic membrane

The amniotic membrane, which had been collected by the above-mentioned procedures (1-2-1 to 1-2-3) and stored, was thawed at room temperature, followed by sufficiently washing with a sterilized phosphate buffer solution (PBS) in a dish. After washing, the amniotic membrane was stored in a 0.02% EDTA solution (Nacalai tesque) at 37°C for 2 hours, and then the epithelium was mechanically scraped off by using a cell scraper (Nunc, USA). The thus obtained amniotic membrane without the epithelium was used as a substrate for the following cell culture.

### 1-5-2 Preparation of epidermal keratinocytes

### 1-5-2-1 1 Collection of skin

A site to be collected is disinfected with disinfectant such as povidone iodine prophylactically in advance and antifungal agent is externally applied thereto, followed by collecting a small skin piece in accordance with skin biopsy.

### 1-5-2-2 Serum free culture of epidermal keratinocytes

Fatty tissue and dermis are removed as much as possible from the skin piece with scissors and washed with Dulbecco's phosphate buffer (PBS) several times. The skin piece is sterilized by immersing it in 70% ethanol for one minute. The skin piece is washed with PBS, then cut into a strip shape with the size of about 3 mm width × 10 mm length, soaked in Dispase solution (Dispase II, Goudou Shusei, 250 units/ml, Dulbecco's Modified MEM culture medium; DMEM) and stood still overnight (18 to 24 hours) at 4°C. On the following day, by using forceps, epidermis is peeled off from dermis. The peeled dermis is subjected to fibroblasts culture. The peeled epidermis is washed with DMEM, then washed with PBS, then soaked into 0.25% trypsin solution and treated at 37°C for 10 minutes. The epidermis is transferred to a plastic dish containing a trypsin neutralization solution, is disentangled by using forceps, and transferred to 50 ml sterilization tube. PBS is added so as to adjust a suspending solution of epidermal keratinocytes. The number of cells is counted and the cells are subjected to centrifugation at 1000 rpm for 5 minutes, so that the cells are precipitated. Supernatant is sucked and the cells are suspended in a MCDB 153 culture medium that is a serum free culture medium, which is seeded at the rate of 2 to 3 × 10⁶ cells/10 ml culture solution for each 100 mm dish coated with collagen (ASAHI TECHNO GLASS CORPORATION, type I collagen coated dish; 4010-O10). On the following day, the culture solution is exchanged, and later than that day, the culture solution is exchanged every other day. At the time when the cell density becomes about 70% to 80%, subculture is carried out.

### 1-5-3 Preparation of fibroblast

After peeled dermis is washed with DMEM, it is cut into strips with the size of 1 to 2 mm × 1 to 2 mm by using a surgical knife. The cut dermis strip is brought into close contact with a dish coated with type I collagen at intervals of about 1 cm. Then, the dermis is stood still in a CO₂ incubator for 30 minutes so as to be brought into close contact the dish completely. Thereafter, about 5 ml of DMEM culture medium containing 10% fetal bovine serum is added and stood still for seven days. On day 7, initial exchange of the culture solution is carried out. It is confirmed that fibroblasts are migrated from the dermis strip. At the stage when cells are proliferated and migrated to 5 mm vicinity of the dermis strip, subculture is carried out. The dermis is washed with PBS, and then 3 ml solution containing 0.125% trypsin and 0.05% EDTA is added and treated at 37°C for three minutes. After it is confirmed through a microscope that cells are detached from the bottom surface of the dish, 3 ml trypsin inhibitor is added and the cells are collected and transferred to 50 ml tube. By using PBS, remaining cells are collected and subjected to centrifugation at 1000 rpm for five minutes, so that cells are precipitated. The supernatant is sucked, and then a DMEM culture medium containing 10% fetal bovine serum is added so as to adjust a cell suspending solution, which is seeded on a cell culture dish. The cell density of subculture is about 1 : 3. The cells are cryopreserved appropriately. As a cryopreservation solution, 10% glycerol, 20% FCS and 70% DMEM are used, and stored in liquid nitrogen.

### 1-5-4 Preparation of neutralized collagen gel

A neutralized collagen solution (final concentration of collagen: 1 mg/ml) is produced at 4°C by using one volume of 0.1N NaOH, one volume of 8 times concentration DMEM, ten volumes of 20% FCS/DMEM to six volumes of type I collagen solution (cell matrix type 1A: 3 mg/ml: Nitta Gelatin Inc.). One ml each of the neutralized collagen solution is dropped into 24 mm diameter culture insert (Corning-Costar) and stood still at room temperature for 10 minutes so as to be gelled. Fibroblasts in a logarithmic growth phase, which has been prepared in advance (cells are subjected to Dispase treatment to peel off epidermis and the remaining dermis is subcultured for 5-10 generations by an outgrowth method, and thus the subcultured cells are obtained and used) are adjusted to the concentration of 5×10⁵ cells/ml and 10% FCS/DMEM. This cell suspension (2 volumes) is mixed with the neutralized collagen solution (8 volumes) so as to prepare a neutralized collagen solution containing cells (final concentration of collagen: 0.8 mg/ml). To each culture insert, 3.5 ml each of this solution is added, and the culture insert is stood still in a CO₂ incubator (37°C, 5% CO₂). After 30 minutes, it is confirmed that the solution is gelled. Thereafter, 10% FCS/DMEM is added so that gel is soaked therein (3 ml is added to the inside of the culture insert, and 3 ml is added to the outside of the culture insert) and static culture is carried out for five days. On day 2 after start of culture, the gel starts to shrink. The proliferation of fibroblasts can be observed under phase contrast microscope.

### 1-5-5 Adhesion of amniotic membrane

On day 5 after start of culture, the bottom surface of the collagen gel is brought into close contact with membrane but the upper part of the collagen gel is shrunk to the thickness of 2 to 3 mm. The preserved amniotic membrane (amniotic membrane prepared by the same procedure described in 1-3-1, from which epithelium has been removed) is washed with PBS twice and then washed with a culture solution of keratinocytes once. The amniotic membrane is transferred to a culture insert with the side of parenchymal cells facing downward and brought into close contact with collagen gel by using forceps. By using forceps, the collagen gel is expanded so that wrinkles are not generated and the periphery of the amniotic membrane is brought into close contact with the side wall of the culture insert, which is transferred to the inside of a CO₂ incubator and stood still at 37°C for 30 minutes.

### 1-5-6 Seeding of keratinocytes

The keratinocytes prepared in 1-5-2 are detached from the dish by using trypsin - EDTA and collected. The keratinocytes are subjected to centrifugation at 1000 rpm for five minutes to remove the supernatant. The cells are suspended so that the concentration becomes 200 million cells /0.25 ml. The cell suspension (0.25 ml) is seeded on the amniotic membrane inside the culture insert, transferred to a CO₂ incubator and stood still in the incubator for 1.5 to 2.0 hours so that keratinocytes are brought into close contact with the amniotic membrane. Thereafter, 1 ml of medium for proliferating epidermal cells is gently added to the inside of the culture insert and further 1 ml of the medium is added to the outside of the culture insert. On the following day, a culture medium for proliferating epidermal cells is gently added to the inside of the culture insert and 1 ml of the medium for proliferating epidermal cells is added also to the outside of the culture insert.

### 1-5-7 Culture under vapor phase conditions

On day 3 after seeding of epidermal cells onto amniotic membrane, air exposure (air lifting) is carried out. Sterilized filter paper is set in a maintaining vessel for air exposure, a stratifying medium is added so that the filter paper is soaked (about 9 ml). The culture solution inside the culture insert is carefully removed and the culture insert is transferred to the filter paper and cultured in a CO₂ incubator. The culture solution is exchanged every other day. By air exposure for 7 to 14 days, a three-dimensional cultured skin is completed. The stratifying culture medium is prepared as follows. Dulbecco's Modified MEM culture medium: F-12 culture medium = 1:1, calcium concentration; 1.95 mM, monoethanolamine; 0.1 mM, O-phosphoethanolamine; 0.1 1 mM, insulin; 5 ug/ml, hydrocortisone; 0.4 ug/ml, L-glutamine; 4mM, Adenin; 0.18 mM, transfferin; 5 ug/ml, selenious acid; 53 nM, triiodothyronine; 20pM, serine; 1 mM, choline chloride; 0.64 mM, linoleic acid; 2 ug/ml, FCS; 2%.
The cultured epidermal sheet obtained by the above-mentioned operation can be easily detached from the bottom surface of the dish or from a collagen matrix. Since the sheet produced by a conventional technique may shrink, it is necessary to use a chitin film (BESCHITIN W) as a support medium. Furthermore, the conventional sheet is often broken. However, according to the above-mentioned method, a strong sheet is prepared and shrinkage of the sheet is not observed, it is not necessary to use a support.
When a cultured epidermal sheet is produced by the above-mentioned method, on day 7 after exposure to the air, epidermis had 5 to 8 layers and the formation of horny cell layer was observed. The cultured epidermal sheet had substantially the same structure as the normal human skin. The histological findings of the stratified keratinocytes shows a cell construct including one layer of basal cell-like cells and 5 to 8 layers of cells stratified and differentiated on the basal cell-like cells. When a cultured epidermal sheet is produced by using cells, which have been subcultured for three generations, at about fourth week following the collection of the skin, the cultured epidermal sheet can be used. The cultured area is increased to several thousand times according to calculation.
Produced cultured epidermal sheet can be frozen by using a small amount of stock solution with or without a carrier. Specifically, firstly, the sheet is cryopreserved in a -80°C freezer, and on the following day, it is preserved in ultra-cold -150°C freezer. By preservation at -150°C, the shape of the sheet can be maintained for a long term. Actually, sufficient treatment effect can be obtained. Besides, the sheet can be preserved at 4°C by using a stock solution used for storing biomedical tissue. In this case, it is desirable that antioxidant is added.

### 1-5-8 Attachment of adhesive component

After the cultured epidermal sheet was taken out from the collagen matrix, the surface opposite side to the surface on which the cell layer had been formed was subjected to air drying by blowing air so as to be in a semidry state. Next, the surface of the semidry amniotic membrane was immersed in a mixed solution including fibrinogen, thrombin and aprotinin (prepared by the procedure similar to the procedure described in 1-2-5), followed by air drying so as to be in a semidry state. During this series of operation, the surface on which cells were cultured was maintained in a wet state. As a result of the above-mentioned operation, a sheet-shaped composition (adhesive attached-cultured epithelial sheet) was obtained.

### 2. Transplantation experiment using adhesive components attached sheet

### 2-1 Application to pig sclera

The adhesive property of the adhesive components attached-epithelium containing amniotic membrane sheet (1-2-5) was examined when the sheet was applied to the pig sclera.
Tissue obtained from the eye of a pig that had been sacrificed for foods (about 6-10 hours had passed after the death) was used. The conjunctiva was excised from the pig eye with scissors so as to expose the sclera (a bare state). At the time of treating pterygium, in general, the abnormal conjunctiva was peeled off and the site from which the abnormal conjunctiva had been removed was covered with the normal conjunctiva. The pig eyes that have been subjected to the above-mentioned treatment are widely used as a clinical model of pterygium.
As a transplantation sheet, adhesive components attached-amniotic membrane sheet with epithelium (1-2-5) was used. The method of applying the transplantation sheet was as follows. First of all, moisture on the surface of the sclera of the pig eye that had been subjected to the above-mentioned treatment was wiped off by using a cotton swab so as to make the tissue semidry. Note here that the level at which fine water drops were not observed by visual inspection was made to be a reference level. The adhesive components attached-amniotic membrane sheet with epithelium that had been prepared in 1-2-5 was put on this sclera with the side on which adhesive components were attached facing downward in a state of dry state. Next, by using forceps, the sheet was slightly pressed and the entire region was brought into contact with the sclera so carefully that bubbles do not enter. By the operation mentioned above, the adhesive components attached on the sheet became wet with a very small amount of moisture remaining on the sclera, so that the sheet and the tissue were adhered to each other.
One week after the sheet was placed, the following two experiments were carried out so as to evaluate the adhesive property.
(1) Shift test: Force is applied to the sheet in the horizontal direction by pressing the sheet with a cotton swab. In this state, it was examined whether or not the sheet was shifted from the tissue.
(2) Stretch test: Force is applied to the sheet in the vertical direction by holding the sheet with forceps. In this state, it is examined whether or not the sheet was peeled off from the tissue.
As a result of the examination, the sheet was neither shifted nor peeled off. Excellent adhesive property was observed.

Subsequently, in order to examine the relationship between the amount of adhesive components and the adhesive property, a plurality of sheets having different amounts of attached adhesive components were prepared and transplanted according to the above-mentioned procedure and each adhesive property was evaluated. The results of the evaluation are shown in the table in Fig. 2. The amount of fibrinogen in the table indicates the amount of attached fibrinogen per 1 cm² of the sheet. Furthermore, "+" in the table indicates that the sheet was neither shifted nor peeled off; and "-" indicates that the sheet was peeled off ("-" was also given in the case of only a small amount of shift). The attached amount of thrombin and the attached amount of aprotinin were adjusted so that the ratios (attached amount of fibrinogen: attached amount of thrombin : attached amount of aprotinin) of the attached components in all the test group were the same.
As shown in the table of Fig. 2, when the attached amount of fibrinogen is not less than 0.5 mg/cm² (attached amount of thrombin: 12 µg/cm², attached amount of aprotinin: 0.12 KIU/cm²), the sheet is neither shifted nor peeled off from the tissue and a very excellent adhesive property is observed. That is to say, it was clearly shown that the attached amount of fibrinogen, 0.5 mg/cm², brings about sufficient adhesive property.

### 2-2 Application to rabbit sclera

The adhesive property and effect of the adhesive components attached-amniotic membrane sheet without epithelium (1-3-1) were examined when the sheet was applied to the rabbit sclera.
Firstly, a rabbit was anaesthetized and the conjunctiva (about 1 × 1 cm) of the eye was excised with scissors so as to expose the sclera (a bare state). Thus, a pterygium model was produced. Next, moisture on the surface of the sclera was wiped off by using a cotton swab so as to make the surface of the sclera semidry. The state in which bleeding is stopped and moisture was not observed by visual inspection was made to be a reference state. The drying operation was carried out immediately before the sheet was applied.
On the sclera, the adhesive components attached- amniotic membrane sheet without epithelium, which had been prepared in 1-3-1, was placed on the sclera with the side on which adhesive components were attached facing downward in a dry state. Next, the sheet was slightly pressed by using forceps, so that an entire region was brought into contact with the sclera so carefully that bubbles are not included. During the examination, antibiotics (Tarivid ointment) and steroid ophthalmic ointment (Rinderon ointment) were applied to the rabbit once a day.
In order to examine the adhesive property of the sheet and the physiological effect after the operation, the surface was observed by photographing by using a camera (the first, second and fourth week after the sheet had been transplanted). The photographing by a camera was carried out by the following procedure. After the rabbit was anaesthetized, the site covered with the sheet was photographed by using Medical Nikol (Nikon) or Slit Lamp (OLYMPUS). At this time, remaining of the sheet, presence or absence of angiogenesis, and presence or absence of inflammation were observed. Next, one drop of fluorescein was dropped and the presence or absence of the covering of the epithelium on the sheet was observed. Four weeks after the transplantation, the tissue of the site on which the sheet was applied was collected and subjected to immunostaining. Thus, the adhesiveness and remaining of the sheet (amniotic membrane) and remaining of fibrinogen were examined.
As a result of the examination, the sheet adhered to the sclera immediately after the transplantation. On week 1 after the transplantation, in the entire region in which the sheet (amniotic membrane) was transplanted, the formation of the epithelium was observed. On the other hand, elicitation of angiogenesis and inflammation were not observed. Also on week 2 and week 4, the sheet maintained the adhesive state. The formation of epithelium was maintained. Also on week 2 and week 4, angiogenesis and inflammation were not observed. Furthermore, as a result of immunostaining, it was found that on week 4, fibrinogen was biodegraded. On the other hand, the amniotic membrane remained on the sclera. Furthermore, on the amniotic membrane, normal formation of the epithelium was confirmed. The covering of the conjunctiva was also normal. From the above-mentioned results, when this sheet is applied, the following findings were obtained: 1) sufficient adhesive property was obtained; 2) epithelium was normally formed; 3) angiogenesis and inflammation were not induced; 4) fibrinogen attached to the sheet was biologically degraded within four weeks at the latest; and 5) covering of the conjunctiva was normally formed. Therefore, it was found that this sheet was able to exhibit the preferable effect as a transplantation material for reconstructing the ocular surface.

### 2-3 Application to rabbit cornea

The adhesive property and effect of the adhesive components attached-cultured corneal sheet (1-4-6) were examined when the sheet was applied to the rabbit cornea.
In a rabbit, all the conjunctival epithelium having a thickness of 100 µm were removed from 4-mm outside of the limbus by using a crescent knife. By this operation, since the epithelial cells containing corneal epithelial stem cells were lost, it can be thought that artificial exhaustion of the ocular surface stem cells is reproduced. Then, the adhesive components attached-cultured corneal sheet was transplanted into the region slightly inner from the limbus. After the operation, antibiotics (Tarivid ointment) and steroid ophthalmic ointment (Rinderon ointment) were applied to the rabbit once a day.
The sheet adhered to the ocular surface immediately after the transplantation. Also on week 4, the favorable adhesive property was maintained. On the other hand, elicitation of angiogenesis and inflammation were slightly observed.

### 2-4 Application to skin

The adhesive property and effect of the adhesive components attached-cultured epidermal sheet (1-5-8) are examined when the sheet is applied to the rabbit skin. The examination can be carried out by the following procedures.
Firstly, hair is shaved in a part of the back of a rabbit and the epidermis is peeled off from the part. Next, the adhesive components attached- cultured epidermal sheet (1-5-8) is transplanted in the part. After transplantation, the sheet is slightly pressed so as to bring the entire region of the sheet into contact with the part of the back. After operation, an antibacterial drug is externally applied once a day. The change of the adhesive state after transplantation and the change over time of the adhesive state are observed. Thereby, the adhesive property and effect of this sheet can be evaluated.

### Industrial Applicability

The sheet-shaped composition provided by the present invention can be used as, for example, a transplantation material for reconstruction of the tissue. The fields in which the sheet-shaped composition of the present invention is applied includes the fields of ophthalmology, digestive surgery, gynecology, and dermatology.
The sheet-shaped composition of the present invention is excellent in the adhesive property. Therefore, in a short time after application, the sheet-shaped composition adhere to the peripheral tissue and it can be expected that an excellent adhesion state is maintained for a long time. Therefore, even if suturing is not carried out, high therapeutic effect can be expected. Some subjects of application may require higher adhesive force, and it may be appropriate to carry out suturing and the like in such subjects. However, such subjects can be handled by relatively simple and easy means. Thus, burden to doctors and patients can be reduced.

The present invention is not limited to the description of the above embodiments and Examples of the present invention. A variety of modifications, which are within the scopes of the claims and which can be easily achieved by a person skilled in the art, are included in the present invention.
All of the articles, publications of unexamined patent application, and Patent Gazettes cited herein are hereby incorporated by reference.

(1) The production method according to claim 17, wherein the step A comprises the following steps:
   (A-1) preparing cells of biological origin;
   (A-2) seeding the cells of biological origin on the amniotic membrane; and
   (A-3) culturing the seeded cells of biological origin and proliferating them.
(2) The production method according to claim 17, wherein the step A comprises the following steps:
   (A-1) preparing cells of biological origin;
   (B) culturing human fibroblasts in a collagen gel; and
   (C) placing the amniotic membrane on the collagen gel, then seeding the cells of biological origin on the amniotic membrane;
   (A-3) culturing the seeded cells of biological origin and proliferating them.
(3) The production method described in the above-mentioned (1) or (2), wherein the step A-3 is carried out in the absence of different types of animal cells.
(4) The production method described in any of the above-mentioned (1) to (3), the method further comprising the following steps:
   (A-4) after the cells of biological origin are proliferated, bringing the outermost layer into contact with the air.
(5) The production method described in any of the above-mentioned (1) to (4), wherein the step A-3 is carried out by using a serum free culture medium.
(6) The production method described in any of the above-mentioned (1) to (4), wherein the step A-3 is carried out by using a culture medium containing, as a serum component, only serum derived from a recipient.
(7) The production method described in claim 17 or any of the above-mentioned (1) to (6), wherein the cells of biological origin are cells derived from the corneal epithelium, conjunctival epithelium, skin epidermis, hair follicle epithelium, oral mucosal epithelium, iris pigment epithelium, retina pigment epithelium, respiratory tract mucosa epithelium or intestinal tract mucosa epithelium.

## Claims

1. A sheet-shaped composition comprising amniotic membrane having fibrinogen and thrombin attached on a surface thereof.

2. The sheet-shaped composition according to claim 1, wherein the surface of the amniotic membrane to which the fibrinogen and the thrombin are attached is in a dry state.

3. The sheet-shaped composition according to claim 2, wherein the amniotic membrane is in a dry state.

4. The sheet-shaped composition according to any one of claims 1 to 3, wherein aprotinin is attached to the surface of the amniotic membrane in addition.

5. The sheet-shaped composition according to any one of claims 1 to 4, wherein the amniotic membrane is amniotic membrane from which epithelium has been removed.

6. The sheet-shaped composition according to claim 5, wherein a cell layer made of cells of biological origin is formed on the amniotic membrane, and the fibrinogen and the thrombin are attached on the surface of the amniotic membrane opposite side to a surface on which the cell layer is formed.

7. The sheet-shaped composition according to claim 6, wherein the cells of biological origin are cells derived from corneal epithelium, conjunctival epithelium, the skin epidermis, hair follicle epithelium, oral mucosal epithelium, iris pigment epithelium, retina pigment epithelium, respiratory tract mucosa epithelium, or intestinal tract mucosa epithelium.

8. The sheet-shaped composition according to any of claims 1 to 7, wherein an amount of attached fibrinogen is 0.5 mg to 20 mg per 1 cm² of the amniotic membrane.

9. The sheet-shaped composition according to any of claims 1 to 8, wherein an amount of attached thrombin is 1 µg to 1 mg per 1 cm² of the amniotic membrane.

10. The sheet-shaped composition according to any of claims 1 to 9, wherein the thickness is in the range from 10 µm to 200 µm.

11. The sheet-shaped composition according to any of claims 1 to 10, which has an adhesive property with respect to a human tissue and does not need suturing at the time of transplantation.

12. A method of producing a sheet-shaped composition, comprising the following steps:
(a) preparing amniotic membrane; and
(b) attaching fibrinogen and thrombin to a surface of the amniotic membrane.

13. The method according to claim 12, further comprising the following step:
(c) carrying out drying process after the step (b).

14. The method according to claim 12 or 13, further comprising the following step:
(b-1) attaching aprotinin to a surface of the amniotic membrane.

15. The method according to any of claims 12 to 14, wherein the step (a) comprises the following step:
(a-1) removing the epithelium from the amniotic membrane.

16. The method according to any of claims 12 to 15, wherein the step (a) comprises the following step:
(a-2) drying the amniotic membrane.

17. The method according to any of claims 12 to 16, wherein the following step is carried out between the step (a) and the step (b):
(A) forming a cell layer made of cells of biological origin on the amniotic membrane.
